# EUROPEAN PATENT SPECIFICATION

(11) **EP 1 519 957 B1**
(45) Date of publication and mention of the grant of the patent: **04.03.2009**
(21) Application number: 03725585.8
(22) Date of filing: 29.04.2003
(51) Int. Cl.: C07K 16/18

(54) **USE OF HMGB1 IN THE TREATMENT OF TISSUE DAMAGE AND/OR TO PROMOTE TISSUE REPAIR**
DIE VERWENDUNG VON HMGB1 ZUR BEHANDLUNG VON GEWEBEVERLETZUNGEN UND/ODER ZUR UNTERSTÜTZUNG DER GEWEBEWIEDERHERSTELLUNG
UTILISATION DE HMGB1 DANS LE TRAITEMENT DES LESIONS TISSULAIRES ET/OU POUR ACTIVER LA REPARATION DES TISSUS

(30) Priority: 03.07.2002 US 393994 P
(43) Date of publication of application: 06.04.2005
(73) Proprietor: FONDAZIONE CENTRO SAN RAFFAELE DEL MONTE TABOR, 20132 Milano (IT); Provincia Italiana della Congregazione Figli dell'Immacolata Concezione-Istituto Dermopatico dell'Immacolata-IRCCS, 00167 Roma (IT); Centro Cardiologico Monzino S.P.A. - IRCCS, 20138 Milano (IT)
(72) Inventor: BIANCHI, Marco Emilio, I-20132 Milano (IT); PALUMBO, Roberta, I-20132 Milano (IT); COLOGNESI CAPOGROSSI, Maurizio Lab. di Patol. Vascolare, 00167 Roma (IT); LIMANA, Federica Lab. Patologia Vascolare, 00167 Roma (IT); GERMANI, Antonia Lab. di Biol. Vascol.e Terapia Genica, 20138 Milano (IT)
(74) Representative: Capasso, Olga
(86) International application number: PCT/IT2003/000265
(87) International publication number: WO 2004/004763

(56) References cited:
- WO-A-00/47104
- WO-A-01/89526
- WO-A-97/23611
- WO-A-02/074301
- WO-A-02/092004
- FANG WEN-HUI ET AL: "The significance of changes in high mobility group-1 protein mRNA expression in rats after thermal injury." SHOCK (AUGUSTA, GA.) UNITED STATES APR 2002, vol. 17, no. 4, April 2002 (2002-04), pages 329-333, XP009020804 ISSN: 1073-2322
- FAGES C ET AL: "REGULATION OF CELL MIGRATION BY AMPHOTERIN" JOURNAL OF CELL SCIENCE, ESSEX, GB, vol. 113, no. 4, February 2000 (2000-02), pages 611-620, XP001070170
- MULLER SUSANNE ET AL: "The double life of HMGB1 chromatin protein: Architectural factor and extracellular signal" EMBO (EUROPEAN MOLECULAR BIOLOGY ORGANIZATION) JOURNAL, vol. 20, no. 16, 15 August 2001 (2001-08-15), pages 4337-4340, XP002261324 ISSN: 0261-4189
- WANG HAICHAO ET AL: "HMGB1 as a late mediator of lethal systemic inflammation" AMERICAN JOURNAL OF RESPIRATORY AND CRITICAL CARE MEDICINE, vol. 164, no. 10 Part 1, 15 November 2001 (2001-11-15), pages 1768-1773, XP002261325 ISSN: 1073-449X
- WANG H ET AL: "HMG-1 AS A LATE MEDIATOR OF ENDOTOXIN LETHALITY IN MICE" SCIENCE, AMERICAN ASSOCIATION FOR THE ADVANCEMENT OF SCIENCE,, US, vol. 285, 9 July 1999 (1999-07-09), pages 248-251, XP002940163 ISSN: 0036-8075 cited in the application
- YANG HUAN ET AL: "HMGB1 as a cytokine and therapeutic target." JOURNAL OF ENDOTOXIN RESEARCH, vol. 8, no. 6, 2002, pages 469-472, XP009020930 ISSN: 0968-0519
- SCAFFIDI PAOLA ET AL: "Release of chromatin protein HMGB1 by necrotic cells triggers inflammation" NATURE (LONDON), vol. 418, no. 6894, 11 July 2002 (2002-07-11), pages 191-195, XP002261326 ISSN: 0028-0836
- YANG R ET AL: "ETHYL PYRUVATE MODULATES INFLAMMATORY GENE EXPRESSION IN MICE SUBJECTED TO HEMORRHAGIC SHOCK" AMERICAN JOURNAL OF PHYSIOLOGY: GASTROINTESTINAL AND LIVER PHYSIOLOGY, AMERICAN PHYSIOLOGICAL SOCIETY, US, vol. 283, 2002, pages G212-G221, XP008019310 ISSN: 0193-1857
- SIMS C A ET AL: "RINGER'S ETHYL PYRUVATE SOLUTION AMELIORATES ISCHEMIA/REPERFUSION-INDUCED INTESTINAL MUCOSAL INJURY IN RATS" CRITICAL CARE MEDICINE, WILLIAMS AND WILKINGS COMPANY, BALTIMORE, MA, US, vol. 29, no. 8, 2001, pages 1513-1518, XP008019328 ISSN: 0090-3493

## Description

### TECHNICAL BACKGROUND

HMGB1 (High mobility group 1 protein) is both a nuclear factor and a secreted protein. In the cell nucleus it acts as an architectural chromatin-binding factor that binds DNA and promotes protein assembly on specific DNA targets (Bustin, Mol. Cell. Biol., 19, 5237-5246, 1999). Outside the cell, it binds with high affinity to RAGE (receptor for advanced glycation endproducts) (Horiet al., J. Biol. Chem. 270, 25752-25761, 1995), and is a potent mediator of inflammation (Wang et al., Science 285, 248-251, 1999; Abraham et al., J. Immunol., 165, 2950-2954, 2000; Andersson et al., J. Exp. Med., 192, 565-570, 2000). HMGB1 is actively secreted by activated monocytes and macrophages (Wang et al., Science 285, 248-251, 1999), and is passively released by necrotic or damaged cells (Degryse et al., J. Cell Biol. 152: 1197-2006, 2001; Müller et al. EMBO J., 16, 4337-4340, 2001; Falciola et al., J. Cell Biol. 137, 19-26, 1997).

Wang et al. (Science, 285, 248-251, 1999) identified HMGB1 as a late mediator of endotoxin lethality in mice. They showed that monocytes/macrophages stimulated by LPS, TNF or IL-1 secreted HMGB1 as a delayed response. In mice, administration of anti-HMGB1 antibodies attenuated LPS-induced endotoxemia; conversely, injection of HMGB1 caused toxic shock. Moreover, septic patients showed increased serum levels of HMGB1, which correlated with the severity of the infection (US 6,303,321; WO 00/47104).

Subsequently, HMGB1 was also shown to cause acute lung inflammation when administered intratracheally (Abraham et al., J. Immunol., 165, 2950-2954, 2000). Antibodies against HMGB1 decreased lung edema and neutrophile migration, whereas they did not reduce the levels of the other proinflammatory cytokines TNF-alpha, IL-1beta or macrophage-inflammatory-protein-2 (MIP2). Pituicytes, which provide an important link between the immune and the neuroendocrine s-ystems, release HMGB1 in response to specific stimuli like TNF-alpha and IL-1beta, suggesting that HMGB1 also participates in the regulation of neuroendocrine and immune responses to inflammatory processes (Wang et al., Surgery, 126, 389-392, 1999). However, most cells (including lymphocytes, adrenal cells or kidney cells) are not able to secrete HMGB1.

The phenomena investigated by the groups of Wang and Andersson depend from the active (if unconventional) secretion of HMGB1 by specific cells, that respond to specific stimulation by proinflammatory cytokines. HMGB1 secretion requires at least 16 hours after cell stimulation, and thus is a late event in inflammation. The role of secreted HMGB1 is thus to reinforce and prolong inflammation that was initiated by some other event.

In contrast, the authors have now demonstrated that HMGB1 released by necrotic cells can be the initial trigger for inflammatory responses, and that released HMGB1 itself can activate inflammatory cells. HMGB1 release and diffusion can take place in a matter of seconds or minutes, and is thus a very early event in inflammation. The ability of certain cell types to secrete HMGB1 without dying must therefore be a type of molecular mimicry: these cells have evolved the ability to secrete the same molecule whose extracellular presence signals tissue damage.

The authors of the instant invention report that *Hmgb1^{-l-}* necrotic cells have a greatly reduced ability to promote inflammation, proving that the release of HMGB1 can signal the demise of a cell to its neighbors, and tissue damage to rest of the organism. Moreover, apoptotic cells do not release HMGB1 even after undergoing secondary necrosis and partial autolysis, and thus fail to promote inflammation even if not promptly cleared by phagocytic cells. In apoptotic cells HMGB1 is firmly bound to chromatin because of generalized chromatin modification, and is released in the extracellular medium (promoting inflammation) if chromatin deacetylation is prevented. Thus, cells undergoing apoptosis are programmed to withhold the signal broadcast by cells damaged or killed by trauma. Therefore, though HMGB1 is an abundant component of chromatin, it also has a role as a soluble, extracellular protein.

The following succession of events could be envisaged:
Necrosis -> primary HMGB1 release -> inflammatory cell activation -> secretion of cytokines -> further activation and recruitment of inflammatory cells -> secretion of HMGB1 by inflammatory cells -> loop continues with secretion of cytokines and further activation and recruitment of inflammatory cells.

This loop will create a positive feedback, with strong inflammatory responses. The loop must be broken at some point to interrupt inflammatory responses.

Besides triggering inflammation, HMGB1 also activates other systems of tissue protection or repair. It is shown in the present invention that HMGB1 can promote the migration and the proliferation of cells whose function is to repair tissue damage.

Foremost among these cells are stem cells. As an example of stem cell recruitment and proliferation, the authors have demonstrated the effect in vitro and in vivo on vessel-associated stem cells, the mesoangioblasts.

As an example of immediate practical application for HMGB1 as a signal for tissue damage, the authors have investigated the regeneration and functional recovery of myocardial tissue after infarction of the heart.

Advantageously HMGB1 promotes regeneration and functional recovery of infarcted heart. Myocardial infarction leads to loss of tissue and impairment of cardiac performance. One of the major therapeutic goals of modem cardiology is to design strategies aimed at minimizing myocardial necrosis and optimizing cardiac repair following myocardial infarction. Although promising results have been obtained with transplantation and mobilization of bone marrow cells to the area of the infarction (Orlic et al., Circ Res. 27, 1092-1102, 2002), signals that regulate stem cells homing to areas of tissue injury were not well characterized so far.

WO02/074337 discloses that HMGB1 may be active in connective tissue regeneration. Connective tissue cells are not embriologically related to target cells of the instant invention.

WO00/47104 claims a pharmaceutical composition for treating conditions (diseases) characterized by activation of the inflammatory cytokines cascade, as sepsis and obesity, comprising an effective amount of an antagonist or inhibitor of HMG-1.

### DESCRIPTION OF THE INVENTION

It is therefore an object of the instant invention the use of a HMGB1 protein or functional parts thereof, or of a HMGB1 expressing vector, for the preparation of a medicament for the treatment of tissue damage and/or to promote tissue repair and regeneration wherein the tissue regeneration depends from the growth of cells of the same type as those damaged, thus excluding the growth of connective tissue to replace lost tissue. More preferably the tissue is cardiac or skeletal muscle.

In a specific aspect of the invention tissue repair and/or regeneration includes the repair and/or regeneration of areas of necrosis, preferably trauma sites, ischemia sites including infarcted heart, bum sites.

In a preferred aspect of the invention the medicament further comprises an effective amount of an anti-inflammation agent. The expert of the field will select the most appropariate anti-inflammation agent.

In a preferred aspect of the invention the medicament further comprises diluents and/or adjuvants for perfusion at the tissue repair site. Alternatively the composition further comprises diluents and/or adjuvants and/or carriers for intramuscular injection.

In a further preferred aspect of the invention the medicament further comprises stem cells, preferably mesoangioblasts.

It is another aspect of the invention a method to promote stem cell migration and/or proliferation in cell culture comprising the step of exposing such cells to an effective amount of the HMGB1 protein or functional parts thereof. Preferably stem cells are resident cardiac or circulating stem cells.

It is another aspect of the invention a method to promote the proliferation of cardiomyocites in cell culture comprising the step of exposing such cells to an effective amount of the HMGB1 protein or functional parts thereof.

### DETAILED DESCRIPTION OF THE INVENTION

The authors have demonstrated that HMGB1 is released by necrotic cells, but not by apoptotic cells. In such a way, HMGB1 can signal tissue damage, since tissue damage produces unprogrammed cell death (necrosis).

The firts effects of HMGB1 released by necrotic cells (in a matter of seconds or minutes) will be the activation of nearby cells, the loss of barrier function of endothelia, the recruitment and activation of myeloid cells, and the triggering of inflammation. As a more delayed response, HMGB1 (either directly released by necrotic cells, or actively secreted by activated inflammatory cells) will promote migration of certain cell types, including stem cells, to the site of tissue damage, and their proliferation.

Specifically, both adult and embryonic vessel-associated stem cells (mesoangioblasts) proliferate when stimulated with HMGB1. HMGB1 was also able to induce mesoangioblast migration as well as transmigration through an endothelial barrier. *In vivo,* heparin beads containing HMGB1 injected into the tibialis muscle were able to attract meso-angioblasts carrying nuclear lacZ injected into the femoral artery.

*In vivo* data show that HMGB1 promotes regeneration of infarcted hearts. As a matter of fact HMGB 1 induces newly formed myocytes into the infarcted heart. The newly formed tissue is visible by staining with hematoxylin and eosin and expresses cardiac molecular markers such as alpha-sarcomeric actin, connexin 4, MEF2 and others. The newly formed tissue is highly proliferative as seen by the expression of Ki67. HMGB1 thus induces cardiomyocyte proliferation and differentiation in the infarcted heart. Moreover, HMGB1 treated hearts improved myocardial function as tested by echocardiographic and hemodynamic parameters.

The effect of HMGB1 on ischemic heart recovery could be due to recruitment/proliferation of resident cardiac stem cells present in the heart and/or to the recruitment/proliferation of circulating stem cells, and/or to the induction of cardiomyocyte proliferation.

HMGB1-induced cardiac tissue regeneration and its effect on myocardial function recovery is comparable to those observed after stem cell transplantation of hematopoietic stem cells (HSC) injected directly into the heart (Orlic et al., Nature, 410, 701-705, 2001). The invention represents a potential novel approach to be used in the treatment of myocardial infarction.

HMGB1 maybe used alone (or in conjunction with cellular therapy) for all types of tissue regeneration. This represents an advantage compared to stem cell-based regeneration procedures, which require GMP extraction and expansion, are subject to transplant rejection/immune response, require bioethical approval and are not easily packaged into an industrial product.

HMGB1 may have the unique capacity of inducing proliferation of cardiomyocytes and thus therapeutic application in several cardiac pathologies.

HMGB1 may be administered as a recombinant protein injected in the contracting wall bordering the infarct or in heparin beads in the tibialis muscle. However, due to the short mean life of the protein the administration scheme may need high or repeated doses to attain therapeutic effects. Therefore, a gene therapy approach could be advantageously used. A nucleic acid coding for HMGB1 may be carried into cells in appropriate vectors (plasmids, viruses).

Dosage is also a factor to be deeply controlled being HMGB1 an inflammatory molecule. In the current study on ischemia, 200 ng of protein was injected in the anterior and posterior aspects of the viable myocardium bordering the infarct.This dose is around 2000 times lower than the dose required for systemic inflammation.

### FIGURE LEGENDS

The invention will be now described with reference to following Figures:

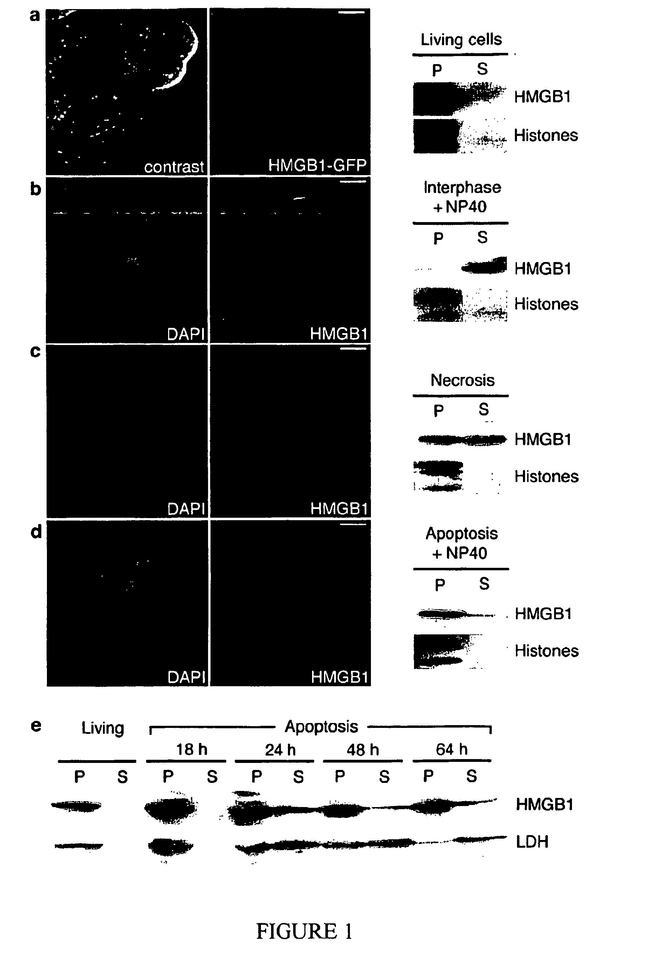

**Figure 1** **Chromatin association of HMGB1 in living and dead HeLa cells.** The medium bathing the cells (S) was analyzed by SDS-PAGE alongside with the cells (P). Histones were visualized by Coomassie staining, HMGB1 by immunoblotting or immunostaining with anti-HMGB1 antibodies, DNA with DAPI. Scale bars, 7.5 µm. a, Living cells expressing HMGB1-GFP, imaged by differential interference contrast and in green fluorescence. b, Interphase cells, after permeabilization. c, Necrotic cells, no permeabilisation. The amount of HMGB1 in the medium was proportional to the number of necrotic cells (about 50%). d, Apoptotic cells, with permeabilization. e, Kinetics of HMGB1 and lactate dehydrogenase (LDH) release from cells undergoing apoptosis and secondary necrosis.

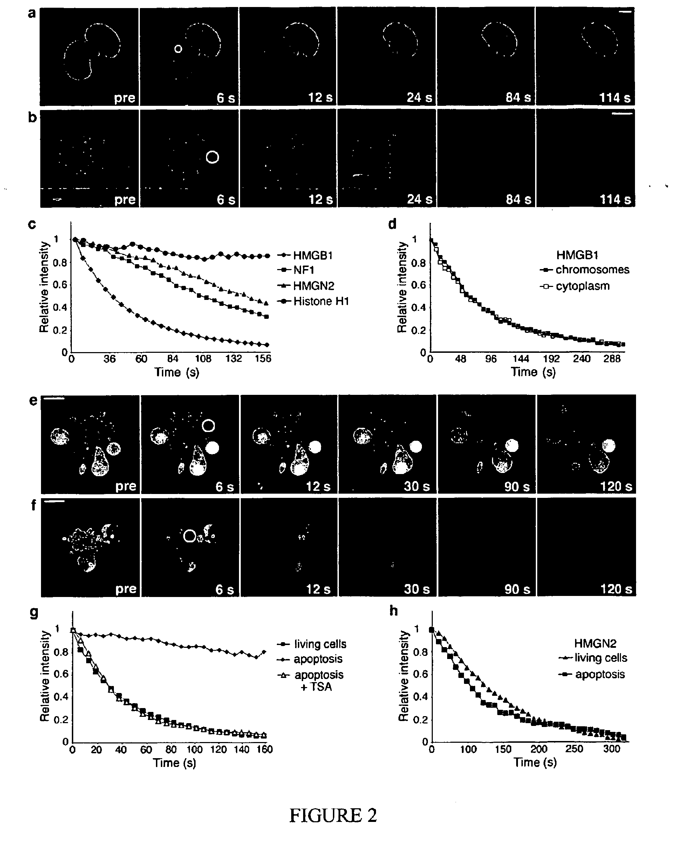

**Figure 2** **HMGB1 dynamics in living and apoptotic cells.** Scale bars, 2.3 µm (a, b) and 3.7 µm (e, f). FLIP imaging (a) and quantitation (c) of HMGB1-GFP in an interphase cell. The area indicated by a circle was repeatedly bleached, and cells were imaged between bleach pulses. A neighbouring cell nucleus was not affected. FLIP imaging (b) and quantitation (d) on a mitotic cell. Bleaching was executed in the cytoplasm (circle), and quantification was done both on a different spot in the cytoplasm, or on a spot on the condensed chromosomes. FLIP imaging in an apoptotic cell (e), and in a cell undergoing apoptosis in the presence of 200 ng/ml TSA (f), and their quantitation (g). h, FLIP quantitation of GFP-HMGN2 in living and apoptotic cells.

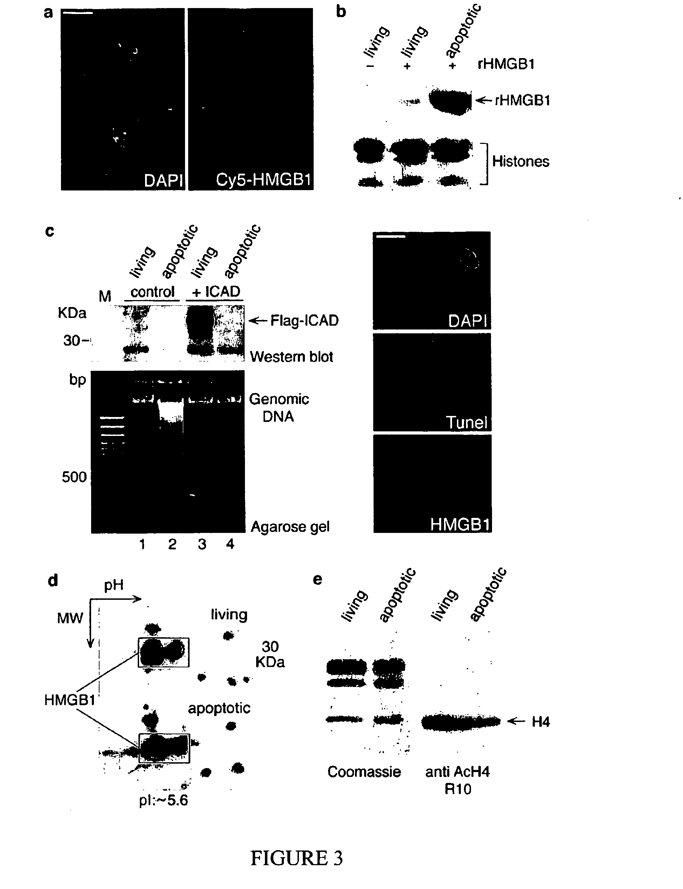

**Figure 3** **Chromatin changes occurring in apoptosis create binding substrates for NMGB1.** Scale bar, 9.5 µm. a,b Bacterially made HMGB1 (either labelled with Cy5, a, or unlabelled, b) binds to the chromatin of apoptotic *Hmgb1*^{*-*/}*⁻* fibroblasts, but not to that of non-apoptotic fibroblasts, as visualized by microscopy (a) or Western blotting (b). Histones were visualized by Coomassie staining. c, DNA fragmentation is not responsible for HMGB1 binding to apoptotic nuclei. HeLa cells (expressing a tagged form of ICAD, or control) were induced into apoptosis (apoptotic, lanes 2 and 4) or were mock treated (living, lanes 1 and 3). ICAD in apoptosis is cleaved by caspases and loses the FLAG tag (Western, anti-FLAG antibodies; lane 4). Agarose gel electrophoresis evidences the internucleosomal cleavage of chromosomal DNA in apoptotic wild type cells (lane 2), and its inhibition in apoptotic ICAD-expressing cells (lane 4). ICAD-expressing apoptotic cells were permeabilised, fixed and stained for DNA, HMGB1 and TUNEL. While all cells are TUNEL-negative, HMGB1 was firmly retained into the nucleus of the cell showing chromatin condensation (in upper right corner). d, Total extracts from about 5 million living and apoptotic HeLa cells were subjected to 2-D electrophoresis and immunoblotted with anti-HMGB1 antibodies. Multiple acetylated forms of HMGB1 are visible, but no difference is detectable between the two samples. e, Histone H4 in apoptotic cells is hypoacetylated. Immunoblotting was performed with R10 antibody (specific for the acetylated forms of H4).

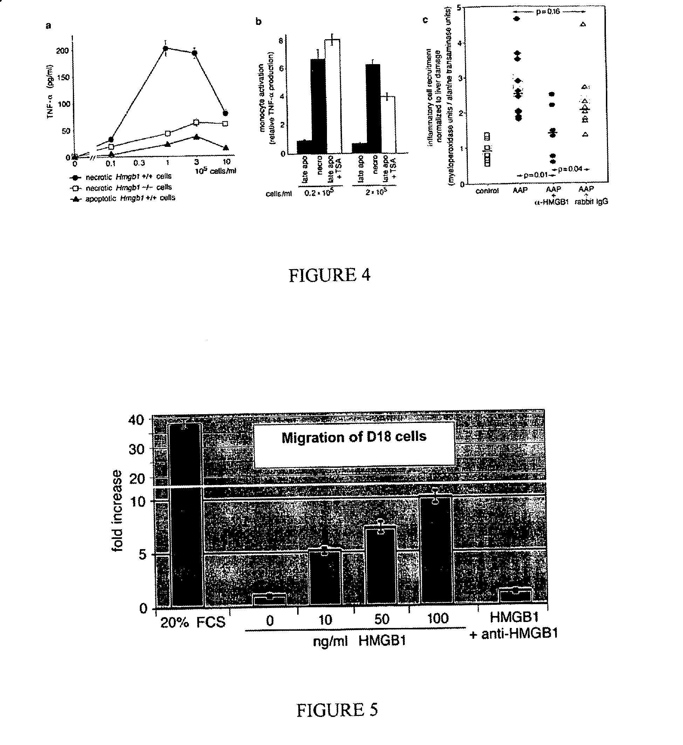

**Figure 4** **HMGB1 release promotes inflammatory responses.** a, Necrotic cells lacking HMGB1 do not elicit the production of the proinflammatory TNF-α cytokine by monocytes. Bars represent standard errors (n=3). b, Apoptotic cells undergoing secondary necrosis and partial autolysis do not promote inflammatory responses, unless HMGB1 is mobilized by treatment with TSA. The experiment was repeated 3 times in duplicate, with 2 different amounts of apoptotic cells to ensure linearity in TNF-alpha production. Values are normalized to a value of 1 for the amount of TNF-alpha added after challenge with 0.2 x 10⁵ apoptotic cells. c, Anti-HMGB1 antibodies reduce inflammation in liver injured by acetaminophen (AAP) overdose. Liver injury (alanine transaminase activity in serum) and inflammatory cell recruitment (myeloperoxidase activity in total liver extracts) was assed after 9 hours. MPO/ALT ratios indicate inflammation normalized to liver damage. Each point represents one mouse, the bar indicates the median value, and the grey shade indicates the area included within average ± standard error. Pairwise comparisons (Mann-Whitney test) between the groups of mice are indicated by the arrows.
**Figure 5** **HMGB1 chemotactic assay on staminal cells.** Chemotaxis assays were performed using modified Boyden chambers. The value of 1 corresponds to the number of cells migrating in the absence of any stimulator (random cell migration). The data represent the mean ±SE. The statistical significance of the result is P<0.0001 in an ANOVA model. Treatment with HMGB1 plus anti-HMGB1 gave results that did not differ statistically from the unstimulated control. Treatment of D18 cells with the anti-HMG1 antibody alone, or an unspecific antibody, also were indistinguishable from the unstimulated control.

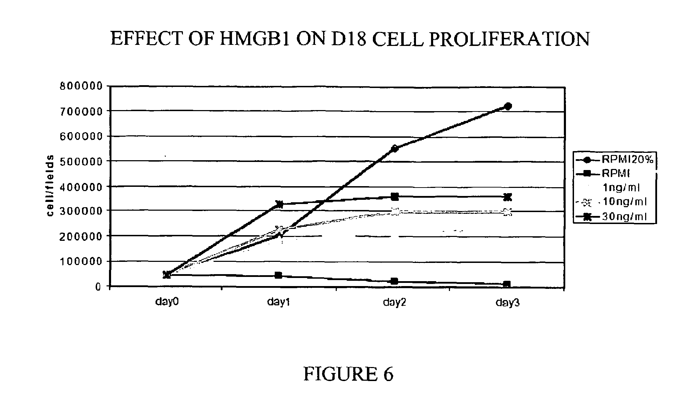

**Figure 6** **Growth curve of staminal cells in the presence of HMGB1.** 5x10⁴ D18 cells were plated in 3 cm wells and grown for 24 hours at 37°C in 5% CO₂ in RPMI supplemented with 20% fetal bovine serum (FBS). Medium was then replaced with RPMI (no serum) for 16 hours. Subsequently the medium was replaced with fresh media containing RPMI alone, RPMI plus 20% FBS, and RPMI with HMGB1 at the indicated concentrations (no serum). Cells were harvested at the indicated times (days 1, 2 and 3) and counted with hemacytometer. Cells in RPMI alone did not divide, while cells in RPMI plus HMGB1 divided actively for at least 24 hours, and then more slowly due to nutrient exhaustion. At day 3, nonetheless, all plates with D18 cells stimulated with HMGB1 (all concentrations) contained some dividing cells, as evaluated by microscopic inspection.

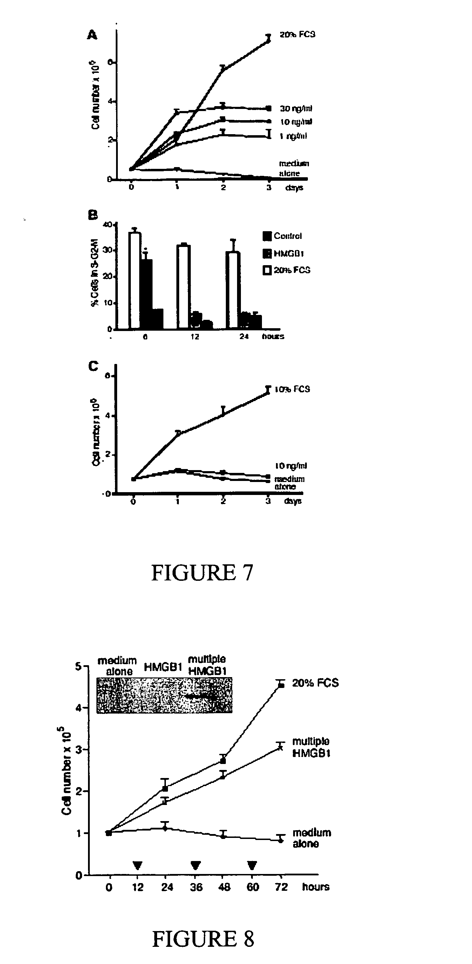

**Figure 7** **Effect of HMGB1 on embryonic mesoangioblast proliferation.** (A) D16 cells were grown in RPMI medium containing no addition, HMGB1 at the indicated concentrations, or 20% FCS. HMGB1 induced cell proliferation at all concentrations tested, but the cell number reached a plateau after 48 hours. Each point represents the mean±SD (n=3). The experiment was repeated 3 times. **(B)** D16 cell division was analyzed by FACS: after 6 hours in the presence of 30 ng/ml HMGB1 the DNA content increases, but returns to the normal diploid content after 24 hours. The asterisk indicates statistical significance (p<0.001). **(C)** 3T3 fibroblasts (treated as the D16 cells in panel A) do not divide in the presence of HMGB1.
**Figure 8** **HMGB1 restimulation prolongs mesoangioblast proliferation.** D16 cells were placed at time 0 in RPMI medium containing 30 ng/ml HMGB1; a similar amount of HMGB1 was also added at the times indicated with a triangle. Multiply-stimulated D16 cells kept growing. Each point represents the average±SD of two experiments performed in duplicate. Inset panel: Western blot of HMGB1 in the medium bathing D16 cells 48 hours after the beginning of the experiment. HMGB1 was still present in the medium of restimulated cells but not in the medium of cells stimulated once at time 0. This experiment was repeated 2 times with similar results.

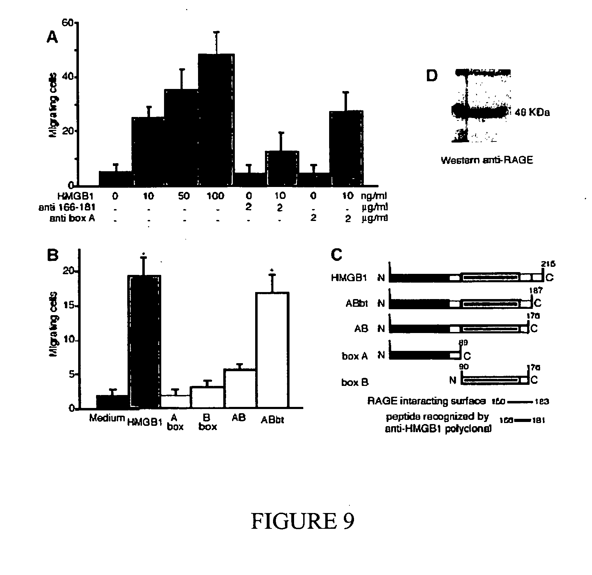

**Figure 9** **HMGB1 has chemotactic activity on embryonic mesoangioblasts. (A)** D16 cells were subjected to chemotaxis assays in Boyden apparatuses with 10, 50, or 100 ng/ml HMGB1. Data represent the average±SD of four experiments performed in duplicate; the effect of increasing HMGB1 concentrations is highly significant (p<0.001 in ANOVA analysis). Addition of anti-HMGB1 antibodies recognizing the peptide 166-181 significantly reduced the chemotactic response (p<0.05 in comparison to the sample without antibody), while the addition of monoclonal anti-boxA antibodies had no effect. **(B)** Chemotactic activity on D16 cells of various HMGB1 fragments (all at 10 ng/ml). Full length HMGB1, boxes A and B, the didomain AB, and tailless HMGB1 (ABbt) are represented in (C). ABbt has a chemotactic effect comparable with full length HMGB1 (p<0.05 of proteins vs medium alone). In contrast, boxes A and B and the AB didomain have no significant chemotactic activity. Bars represent the average±SD of three experiments performed in duplicate. (D) Western blot with anti-RAGE antibodies on total D16 cell extract.

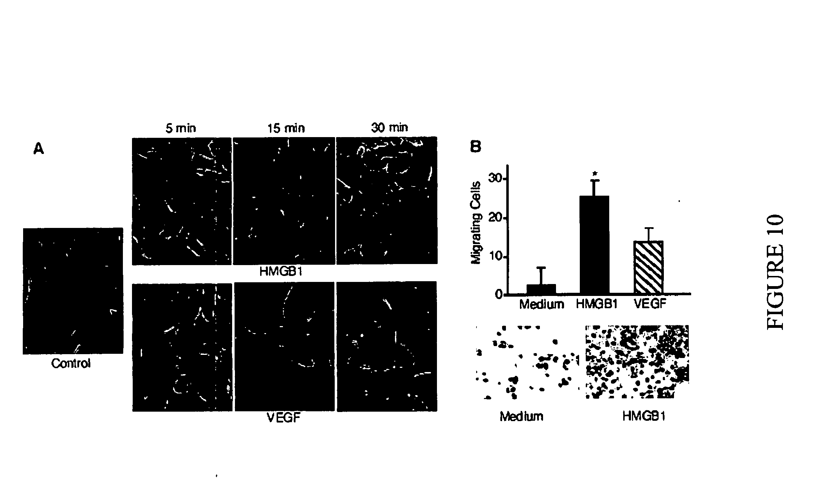

**Figure 10** **HMGB1 induces the transit of mesoangioblasts through an endothelial monolayer. (A)** Bovine primary endothelial cells (BAEC) grown to confluence on glass coverslips were exposed to HMGB1 or VEGF and stained with FITC-labeled phalloidin to visualize actin fibers. Both treatments determined the formation of stress fibers, and the separation of cells form each other. **(B)** Embryonic mesoangioblasts were placed in the upper compartment of Boyden apparatuses; the lower chambers contained RPMI alone (medium), RPMI plus 100 ng/ml HMGB1 or RPMI plus 10 ng/ml VEGF; chambers were separated by a confluent endothelial cell monolayer grown on polycarbonate filters. HMGB1 significantly stimulated D16 transmigration (p<0.01). Bars represent the average±SD of three experiments performed in duplicate. The panels above the bar graph show D16 cells stained with Giemsa, after migration towards medium alone or RPMI plus HMGB1.

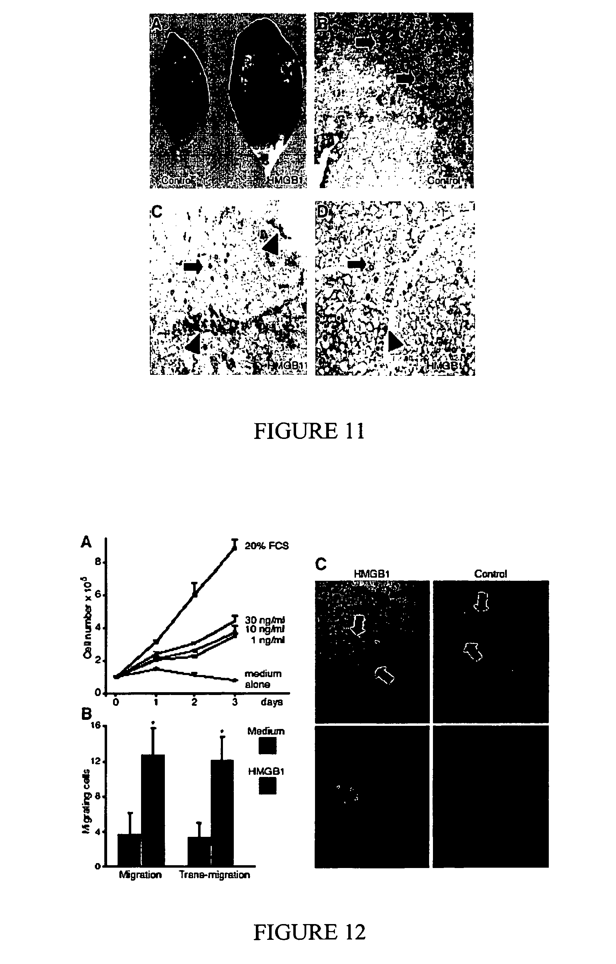

**Figure 11** **HMGB1 attracts mesoangioblasts in vivo.** D16 cells were first transduced with a lentiviral vector encoding nuclear LacZ and then injected through the femoral artery of mice where heparin beads (either loaded with HMGB1 or unloaded) had been injected in the *tibialis anterior* muscle. Mice were then sacrificed after 24 hours. **(A)** *Tibialis anterior* muscles injected with HMGB1-loaded and control beads. **(B, C, D)** Criosections of muscles treated with control heparin beads (B) or HMGB1-coated heparin beads (C, D). Arrows indicate the beads. Sections were stained with X-gal and mesoangioblasts (arrowheads) appear blue. Mesoangioblasts were found in large clusters (C) or as isolated cells (D) only in muscles injected with HMGB1-coated beads.
**Figure 12** **Effect of HMCB1 on adult mesoangioblasts. (A)** Mesoangioblasts of the G1 clone, obtained from mouse bone marrow, were grown in RPMI medium containing of 1, 10, or 30 ng/ml HMGB1. For comparison G1 cells were also grown in RPMI medium alone or RPMI plus 20% FCS. **(B)** Migration of G1 cells towards the lower chamber of Boyden apparatuses containing RPMI (medium) or RPMI plus 10 ng/ml HMGB1 (HMGB1). In the "migration" experiment the chambers were separated by a filter, in the "transmigration" experiment the chambers were separated by a filter overlayed with a monolayer of endothelial cells. Each bar represents the average±SD of three experiments, and the results are statistically significant (p<0.05). **(C)** G1 cells were labeled with DiI and then injected through the femoral artery of mice where heparin beads (either loaded with HMGB1 or unloaded) had been injected in the *tibialis anterior* muscle. Upper, phase contrast; lower, fluorescence. G1 cells (red fluorescence) migrate in the vicinity of HMGB1-loaded cells; no G1 cells are detected near control beads.

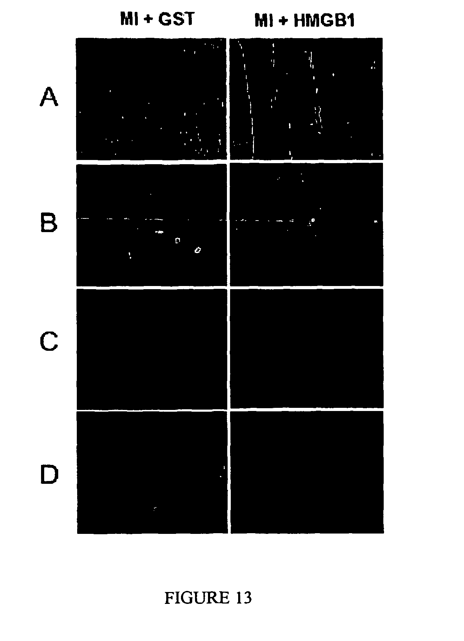

**Figure 13** **HMGB1 induces the formation of new cardiomyocytes** *in vivo* **(A)** Hematoxylin and eosin staining of the infarct zone in GST and HMGB-1 treated hearts. A significant modification in tissue organization was observed in HMGB1 injected hearts. **(B)** KI67 immunostaining (green fluorescence) reveals a number of positive cells in HMGB1-injected hearts. **(C)** α-sarcomeric actin immunostaining (red fluorescence). Blue fluorescence, Hoechst labeling of nuclei. α-sarcomeric actin expressing cells are detected in HMGB1 treated hearts. **(D)** MEF-2 immunostaining (green bright fluorescence).

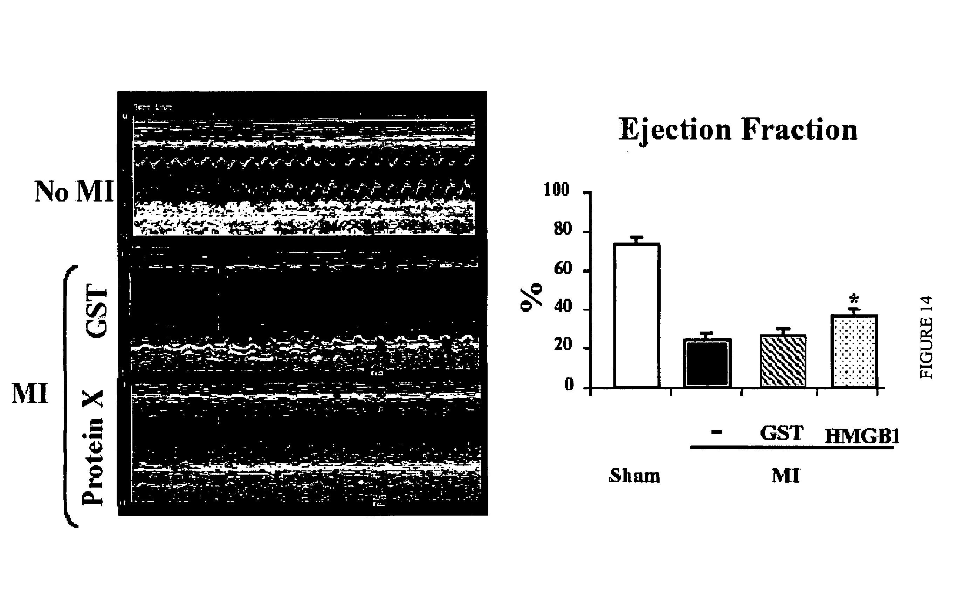

**Figure 14** **HMGB1 treatment improves myocardial function: echocardiographic studies.** Representative echocardiography of control (non-infarcted) and infarcted mice (left panel). Ejection fraction (EF) calculated by echocardiography (right panel). Results are means ± standard deviation (SD). *P<0.05 HMGB1 ν*s*. GST.

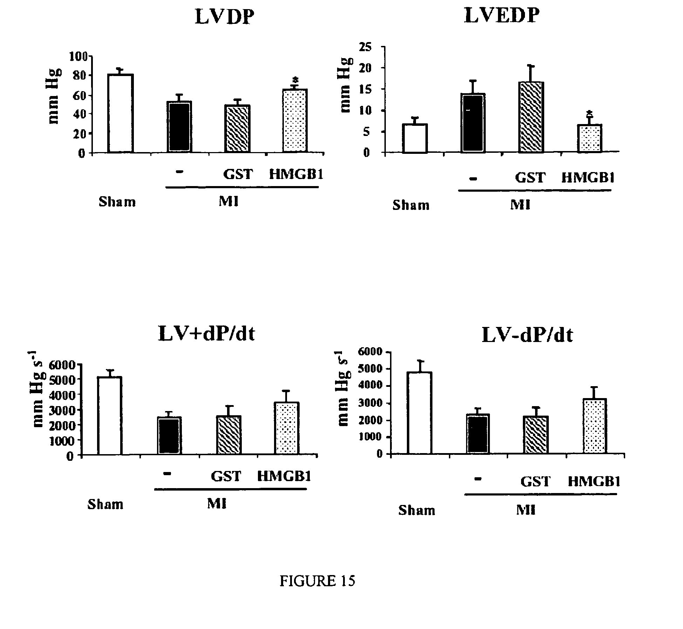

**Figure 15** **HMGB1 treatment improves myocardial function: hemodynamic studies.**. Effects of myocardial infaction (MI) on developed pressure (LVDP), left ventricular end-diastolic pressure (LVEDP), LV+dP/dt (rate of pressure rise) and LV-dP/dt (rate of pressure decay). Results are from sham-operated (sham) and infarcted mice untreated and treated either with GST or HMGB-1. Results are means ± standard deviation (SD). The asterisk denotes statistical significance in Student's t test (p<0.05).

### EXAMPLE 1

### MATERIALS AND METHODS

### Nomenclature

High mobility group proteins have been renamed recently. Previous/alternative names for HMGB1 are High mobility group 1, HMG1, HMG-1, amphoterin, and p30.

### Cloning, expression and purification of HMGB1 protein and fragments

The plasmids HMGB1 and fragments thereof have been described (Müller et al., Biochemistry, 40, 10254-10261, 2001). Protein concentrations were determined spectroscopically using the method of Gill and von Hippel (Analyt. Biochem., 182, 319-326, 1989). The following extinction coefficients have been used for the native protein: Box A: A₂₈₀= 9.98 10³ M⁻¹ cm⁻¹, Box B: A₂₈₀= 1.15 10⁴ M⁻¹ cm⁻¹, AB, AB_{bt} and full-length HMGB1: A₂₈₀= 2.14 10⁴ M⁻¹ cm⁻¹.

### Constructs and cells.

Plasmid pEGFP-HMGB1 was generated by inserting the coding sequence of the cDNA for rat HMGB1 into pEGFP-N1 (Clontech) using the *EcoR*I and *Sac*II restriction sites. pEGFP-Hlc, pEGFP-NF1, pEGFP-HMGN2, and pEF-flag-mICAD were generously provided by A. Gunjan, N. Bhattacharyya, R. Hock, M. Bustin and S. Nagata (Phair and Misteli, Nature, 404, 604-609, 2000; Misteli, et al., Nature 408, 877-881, 2000; Enari et al., Nature 391, 43-50, 1998). HeLa cells and fibroblasts (line VA1, *Hmgb1*^{+/+}*,* and line Cl, *Hmgb1*^{*-*/*-*}) were grown as described (Calogero et al., Nature Genet. 22, 276-280, 1999). HeLa cells were electroporated with pEGFP-HMGB1 and were observed 18 h later. The average amount of HMGB1-GFP in the cell population was between 1 and 3% of HMGB1 (by immunoblotting with anti-HMGB1 antibodies). At the single cell level, the amount of HMGB1-GFP varied at most tenfold between different cells; care was taken to always use for analysis cells with a moderate fluorescence level.

Apoptosis was induced by treating the cells for 16 h with 2 ng/ml human TNF-alpha and 35 µM cycloheximide. Necrosis was induced by treatment for 16 h either with 5 µM ionomycin and 20 µM CCCP, or 6 mM deoxyglucose and 10 mM sodium azide, or by 3 cycles of freezing and thawing.

Three different clones of HeLa cells stably transfected with pEF-flag-mICAD were stained with TUNEL (Apoptosis detection system, Promega), and their chromosomal DNA was extracted and electrophoresed on a 1.5% agarose gel.

Indirect immunofluorescence was performed as described (Degryse et al., J. Cell Biol., 152, 1197-2006, 2001) using an anti-HMGB1 polyclonal antibody (Pharmingen) at 1:1600 dilution, and FITC- or TRIC-conjugated anti-rabbit antibodies (Boehringer) at 1:300 dilution.

### In vivo microscopy and FLIP.

Cells were plated and observed in LabTek II chambers (Nalgene) with an Axiovert 135M microscope (Zeiss). FLIP experiments were carried out on a Leica TCS-SP confocal microscope using the 488 nm excitation line of an Ar laser and detection at 500-575 nm as described (Phair and Misteli, Nature, 404, 604-609, 2000). Cells were bleached (in a spot of 1 µm in radius, 20 mW nominal output, 200-500 ms) and imaged (at 0.2 mW nominal output) at intervals of 6 s.

### Binding of recombinant HMGB1 to chromatin.

*Hmgb1^{-l-}* fibroblasts were treated with 2 ng/ml hTNF-alpha and 35 µM cycloheximide. After 16 h, apoptotic cells were recovered by gentle flushing of the dish. Ten million apoptotic *Hmgb1^{-l-}* fibroblasts and a control population of non-apoptotic ones were resuspended in 50 µl PBS containing 0.32 M sucrose, 0.5% NP-40 and 1 µM bacterially produced HMGB1, either fluorescently labelled with Cy5 (Pharmacia) or unlabelled. Average labelling was 2.3 Cy5 molecules per HMGB1 molecule. After 30 minutes at room temperature, sample cells were mixed and mounted on slides using Vectashield (Vector Laboratories) containing 1.5 µg/ml DAPI, and observed on an Axiophot microscope (Carl Zeiss) with TRITC filter. The two pools of cells incubated with unlabelled HMGB1 were layered onto discontinuous gradients formed by 5 ml of 1.16 M sucrose in PBS and a 6 ml cushion of 2 M sucrose in PBS, and centrifuged at 30,000 g for 90 minutes in a SW27 Beckman rotor. Apoptotic and non-apoptotic chromatin free from membrane debris was recovered from the bottom of the tubes, and applied to a 12% SDS-PA gel. The amount of recombinant HMGB1 bound to apoptotic and non-apoptotic chromatin was determined by immunoblotting using an anti-HMGB1 antibody (Pharmingen) at 1:3000 dilution. Aliquots of apoptotic and non-apoptotic chromatin were also probed with anti-acetyl-histone H4 (R10, a gift from B. Turner), anti-acetyl-Histone H3 (Lys 9, Biolabs) and anti-acetyl-lysine antibody (Biolabs).

*Inflammation assays.* To measure TNF-alphaproduction in vitro, bone marrow was recovered from the hind legs of female C56B16 mice, diluted to 5 10⁶ cells/ml in Optimem and dispensed in 96-well microtiter plates (120 µl per well). Necrotic cells (lysed by 3 cycles of freeze-thawing) or apoptotic cells were added to the indicated final concentration into the wells and incubated at 37°C for 18 hours. TNF-alpha in the supernatant was assayed by ELISA (Quantikine M, R&D Systems). TSA was added at 200 ng/ml together with TNF- alpha, when indicated, and was washed away before mixing the apoptotic cells with bone marrow cells.

To measure inflammation *in vivo,* one day old mice (weighing 1.1± 0.1 grams) were injected intraperitoneally with 20 µl of PBS containing 320 µg of acetaminophen (Sigma), and 320 µg of antibodies (Pharmingen BD) where indicated. After 9 hours the mice were analyzed for serum ALT activity with the GP-Transaminase kit (Sigma) and for MPO activity in liver extracts as described (Kato et al., Am. J. Pathol. 157: 297-302, 2000). Statistical analysis was performed with the non-parametric Mann-Whitney test on MPO/ALT ratios. Similar results were obtained using the t-test on the MPO levels of mice paired so as to minimize the difference in ALT levels.

### RESULTS

HMGB1 is loosely bound to chromatin of both interphase and mitotic cells, and it is rapidly leaked out into the medium when membrane integrity is lost in permeabilized or necrotic cells (Degryse et al. , J. Cell Biol. 152: 1197-2006, 2001; Müller et al., EMBO J., 16, 4337-4340, 2001; Falciola et al., J. Cell Biol. 137, 19-26, 1997). These results suggested that in living cells HMGB1 associates and dissociates rapidly from chromatin. To prove this, HMGB1 was tagged with GFP at its C terminus, forming a chimeric protein that was equivalent to the unperturbed HMGB1 in enhancing the expression of a HOXD9-responsive reporter gene in transfection assays (Zappavigna et al., EMBO J., 15, 4981-4991, 1996). HeLa cells expressing the fusion protein were easily detectable by the uniform green fluorescence of their nuclei. Cells undergoing mitosis showed a diffuse cytoplasmic fluorescence, but also a distinct association of HMGB1-GFP to condensed chromosomes, that lasted throughout M phase (Fig. 1a). When HMGB1-GFP transfected HeLa cells were permeabilized with NP-40, most lost their fluorescence after a few seconds, confirming the loose association of HMGB1 to chromatin. However, a few cells retained a bright fluorescence. From the characteristically fragmented appearance of their nuclei, these cells appeared apoptotic. HeLa cells were then forced to undergo apoptosis by treatment with TNF-alpha and cycloheximide, permeabilized, and immunostained for their endogenous, unmodified HMGB1. Whereas control non-apoptotic cells leaked all HMGB1 into the medium (Fig. 1b,d), the protein was retained within the nucleus of apoptotic cells (Fig. 1d). HMGB1 was mostly retained associated with nuclear remnants even after prolonged incubation and partial autolysis of apoptotic cells, when soluble cytoplasmic proteins like lactate dehydrogenase (LDH) leaked into the extracellular medium (Fig. 1e). HMGB1 and HMGB1-GFP also bound tightly to chromatin in HeLa and 3T3 cells induced into apoptosis by etoposide or H₂O₂ treatment, or apoptosing spontaneously in unperturbed cultures (data not shown). In contrast, HMGB1 dissociated from the chromatin of necrotic cells and leaked to the extracellular medium (Fig. 1c).

To quantify the dynamic properties of HMGB1-GFP within single cells, a technique called fluorescence loss in photobleaching (FLIP) was used (Phair and Misteli, Nature, 404, 604-609, 2000). Repeated bleaching of the same area leads to fluorescence loss from the rest of the nucleus, with kinetics dependent on the overall mobility of the fluorescent protein. If a fraction of the protein pool is at any given time bound to chromatin, the loss of its fluorescence will be slowed. Bleaching of total nuclear HMGB1-GFP was rapidly obtained (Fig. 2a); in contrast, in HeLa cells expressing GFP fusions to chromatin proteins HMGN1 and HMGN2, or transcription factor NF1, fluorescence loss was significantly slower; in cells expressing GFP-histone H1c fusions, fluorescence loss was very limited (Fig. 2c).

The authors also assessed the diffusion rate of HMGB1-GFP associated to condensed chromosomes of living HeLa cells during mitosis. Repeated bleaching of cytoplasmic HMGB1-GFP led to rapid and parallel loss of fluorescence from condensed chromosomes and from the cytoplasm (Fig. 2b,d), proving unequivocally that HMGB1 turns over fast between the chromatin-bound and soluble states.

To the other extreme, HMGB1-GFP appeared almost immobile in apoptotic cells (Fig. 2e,g). The blockade of HMGB1 is specific, since the mobility of GFP-HMGN1, GFP-HMGN2, GFP-NF1 and GFP alone is not reduced in apoptotic cells as opposed to living ones (Fig. 2h and results not shown). Thus, chromatin condensation during apoptosis does not impair protein mobility in general.

*Hmgb1*^{*-*/}*⁻* cells offered us the opportunity to test whether the binding of HMGB1 to apoptotic chromatin was due to alterations of HMGB1, or of nuclei undergoing apoptosis. Embryonic fibroblasts obtained from *Hmgb1^{-l-}* and *Hmgb1*^{*+*/}*⁺* mice

Calogero et al., Nature Genet., 22, 276-280, 1999) were equally susceptible to apoptosis (not shown), indicating that freezing of HMGB1 onto chromatin is a consequence of apoptosis, but not a requisite. *Hmgb1*^{*-*/}*⁻* fibroblasts were treated with TNF-alpha and cycloheximide, and apoptotic cells were recovered from the flask by gentle flushing. This cell population, and a control population of non-apoptotic *Hmgb1^{-l-}* fibroblasts, were permeabilized with detergent and exposed to bacterially produced, Cy5-labelled HMGB1. HMGB1 bound to apoptotic nuclei, but not to non-apoptotic ones (Fig. 3a). This result was confirmed biochemically (Fig. 3b): permeabilized apoptotic and non-apoptotic *Hmgb1*^{*-*/}*⁻* fibroblasts were incubated with bacterially made HMGB1 and fractionated through a discontinuous sucrose gradient. Again, HMGB1 associated to the nuclei from apoptotic cells, but not to the ones from non-apoptotic cells. The experiments described above indicate that, upon apoptosis, chromatin undergoes some chemical or structural transition that makes it susceptible to HMGB1 binding. The nature of HMGB1 itself, whether endogenous or made in bacteria, tagged with fluorophores or fused to GFP, is irrelevant.

The authors next investigated the nature of the chromatin modification allowing the stable binding of HMGB1. Since HMGB1 binds tightly to in vitro reconstructed mononucleosomes (Falciola et al., J. Cell Biol., 137, 19-26, 1997), the authors tested whether the fragmentation of chromatin to oligo- and mononucleosomes that occurs in the later stages of apoptosis would provide stable binding sites for HMGB1. HeLa cells were stably transfected with a construct expressing ICAD, the inhibitor of the CAD nuclease that fragments DNA during apoptosis. HeLa cells overexpressing ICAD underwent apoptosis, but their DNA showed little if any fragmentation (Enari, et al., Nature, 391, 43-50, 1998) (Fig. 3c). HMGB1 bound equally stably to ICAD-expressing, nonfragmented chromatin, and to fragmented chromatin (Fig. 3c). DNA fragmentation therefore cannot account for stable HMGB1 binding in apoptosis.

Alteration of the acetylation status of chromatin was tested next. TSA, a general deacetylase inhibitor, was added to the medium of HeLa cells just prior to the induction of apoptosis: in this case, HMGB1 freezing onto chromatin was suppressed (Fig. 2f,g). This result suggests that hypoacetylation of one or more chromatin components occurs during apoptosis, and favors HMGB1 binding. No difference was seen in the pI or the molecular weight pattern of HMGB1 present in apoptotic and non-apoptotic cells (Fig. 3d), indicating that apoptosis was not changing the acetylation status of HMGB1 itself. In contrast, histone H4 from apoptotic chromatin is notably hypoacetylated in comparison to non-apoptotic chromatin, and H4 hypoacetylation in apoptosis is suppressed by TSA (Fig. 3e).

The authors thus showed that HMGB1 binding to chromatin depends on the viability of the cell, and clearly distinguishes necrotic from apoptotic cells. The differential release of HMGB1 might be exploited as a cue to nearby cells to activate the appropriate responses to unprogrammed and programmed cell death. Unprogrammed death is usually the result of trauma, poisoning or infection, all events that require prompt reaction and damage containment and/or repair. Inflammation is the primary tissue damage response in mammals, and HMGB1 has already been reported to be a mediator of inflammation (Wang et al., Science, 285, 248-251, 1999; Abraham et al. J. Immunol., 165, 2950-2954, 2000; Andersson et al., J. Exp. Med. 192, 565-570, 2000). To test directly whether the release of HMGB1 by necrotic cells can be the immediate trigger for an inflammatory response, the authors challenged wild type bone marrow cells with *Hmgb1^{-l-}* or wild type (+/+) dead fibroblasts. As expected, wild type necrotic cells triggered the production of the proinflammatory cytokine TNF-alpha, whereas wild type apoptotic cells were much less effective (Fig. 4a). Significantly, *Hmgb1^{-l-}* necrotic cells were also rather ineffective in activating monocytes. Purified HMGB1 also elicits TNF-alpha production in this assay (Andersson et al., J. Exp. Med. 192, 565-570, 2000). Thus, this experiment shows that HMGB1 is one of the major diffusible signals of necrosis. On the other hand, it cannot test whether apoptotic cells escape the inflammatory surveillance because they retain HMGB1: apoptotic cells start to leak out cellular components only after several hours, and in vivo they are routinely cleared by phagocytic cells well before this process (termed secondary necrosis) can take place. The authors could nonetheless test whether cells undergoing post-apoptotic, secondary necrosis are able to promote inflammatory responses in monocytes. Wild type, apoptotic fibroblasts were incubated with for 72 hours, until most LDH was released in the extracellular medium; the post-apoptotic cell remnants did not promote a strong inflammatory response in monocytes (Fig. 4b). However, fibroblasts treated with TSA while undergoing apoptosis generated secondarily necrotic cell remnants that promoted inflammation as vigorously as primary necrotic cells killed by freeze-thawing.

The authors could not test whether *Hmgb1^{-l-}* mice have a reduced inflammatory response following tissue necrosis, because these mice survive only a few hours after birth (Calogero et al., Nature Genet., 22, 276-280, 1999). To provide evidence in an animal model for the significance of HMGB1 release, massive hepatocyte necrosis was induced in wild type mice and the inflammatory response was measured. Both in humans and rodents, an overdose of the analgesic acetaminophen (AAP, also known as paracetamol) produces large areas of liver necrosis, concomitant with local inflammation, Kupffer cell activation and the recruitment and sequestration of neutrophils and macrophages into the injured tissue (Lawson et al., Toxicol Sci., 54, 509-516, 2000; Thomas, Pharmacol. Ther., 60, 91-120, 1993). Levels of liver damage and neutrophil sequestration are strictly proportional until most hepatocytes become necrotic between 12 and 24 hours after AAP poisoning (Lawson et al., Toxicol Sci., 54, 509-516, 2000). The authors administered 300 mg/kg AAP with a single intraperitoneal injection to young mice, and 9 hours later estimated liver injury by measuring alanine transaminase (ALT) activity in serum, and inflammatory cell sequestration by measuring myeloperoxidase (MPO) activity in total liver extracts.

One group of mice (n=8) received no AAP, one group (n=10) received AAP alone, another (n=6) AAP and affinity purified anti-HMGB1 antibodies (300 mg/kg), and the last one (n=8) AAP and irrelevant rabbit antibodies (300 mg/kg). All 3 groups injected with AAP had elevated ALT levels in comparison to sham-treated controls, but the differences between the 3 treated groups were not statistically significant.

Thus, antibodies do not protect against liver damage, at least at the onset of the inflammatory response. The authors then used the MPO/ALT ratio to compare inflammatory cell recruitment, normalized to the level of liver damage (Fig. 4c). Anti-HMGB1 antibodies were effective in reducing inflammation following AAP-induced liver necrosis: these mice showed a significantly reduced MPO/ALT ratio (1.5± 0.3) both in comparison to mice injected with AAP alone (2.7± 0.3; p<0.05), and to mice injected with AAP and preimmune rabbit IgGs (2.4± 0.3; p<0.05). No HMGB1 can derive from activated monocytes and macrophages in our experiment, because HMGB1 secretion from inflammatory cells requires at least 16 hours (Wang et al., Science, 285, 248-251, 1999; Abraham et al. J. Immunol., 165, 2950-2954, 2000; Andersson et al., J. Exp. Med. 192, 565-570, 2000). Thus, HMGB1 acts as an immediate trigger of inflammation, as well as a late mediator of inflammation as previously described (Wang et al., Science, 285, 248-251, 1999).

In conclusion, the authors have shown that the passive release of an abundant chromatin component can serve as a diffusible signal of *unprogrammed* death, that can be used as a cue to nearby cells. Core histones, though more abundant, would probably not be good signals of necrosis, as they remain anchored to the insoluble chromatin of necrotic cells. Apoptotic cells are not the result of a present and immediate danger and do not trigger inflammation in physiological conditions. They retain nuclear components until cleared by macrophages or nearby cells that act as semi-professional phagocytes, that they attract and activate by displaying "eat me" signals (Ren and Savill, Cell Death Different., 5, 563-568, 1998). However, apoptotic cells that escape prompt clearance undergo secondary necrosis, lead to an increased level of nuclear autoantibodies (Scott et al., Nature, 211, 201-211, 2001), and have been proposed to play an important pathogenetic role in autoimmune diseases, such as lupus (Herrmann et al., Arthritis Reum., 41, 1241-1250, 1998). Thus, the retention of HMGB1 by apoptotic cells undergoing secondary necrosis represents an additional safeguard against confusing necrotic and apoptotic cells.

### EXAMPLE 2

The authors also demonstrated that HMGB1 has chemotactic activity on D18 mouse mesoangioblasts derived from fetal aorta cells (Minasi et al., Development. 129, 2773-83, 2002). Mesoangioblastic stem cells can be derived from mouse fetal aorta cells but also from umbilical chord cells, peripheral blood vessels and bone marrow ckit+ cells in post-natal mice. Once derived from these original cell populations with a proprietary method (described in Minasi et al., Development. 129, 2773-83, 2002), mesangioblastic cells, of which D18 are an example, are 'naturally' immortalized (they grow indefinitely). D18 cells serve as precursor of the following mesodermal tissue types: bone, cartilage, skeletal smooth and cardiac muscle, endothelial cells, monocytes, macrophages and osteoclasts. They are also capable of generating hepatocytes and neurons. Clone D18 was deposited according to the Budapest Treaty at CBA, Centro Biotecnologie Avanzate, Genova, Italy, N. PDO2005). Chemotaxis assays were performed using modified Boyden chambers. The value of 1 corresponds to the number of cells migrating in the absence of any stimulator (random cell migration).

The data represent the mean ±SE. The statistical significance of the result is *P*<0.0001 in an ANOVA model. Treatment with HMGB1 plus anti-HMGB1 gave results that did not differ statistically from the unstimulated control. Treatment of D18 cells with the anti-HMGB1 antibody alone, or an unspecific antibody, also were indistinguishable from the unstimulated control.

Stem cells are expected to proliferate at the site where tissue repair must take place. The authors then tested whether HMGB1 could also stimutate stem cell proliferation. Stem cells in RPMI with no serum did not divide, while cells in RPMI with no serum but in the presence of HMGB1 divided actively for at least 24 hours, and then more slowly due to nutrient exhaustion. At day 3, nonetheless, all plates with D18 cells stimulated with HMGB1 (at all concentrations) contained some dividing cells, as evaluated by microscopic inspection.

Cells in RPMI alone did not divide, while cells in RPMI plus HMGB1 divided actively for at least 24 hours, and then more slowly due to nutrient exhaustion. At day 3, nonetheless, all plates with D18 cells stimulated with HMGB1 (all concentrations) contained some dividing cells, as evaluated by microscopic inspection (Fig. 6).

The experiment has been repeated with mesangioblasts derived from adult mouse capillaries (stem cells from adult), showing the same proliferation inducing effect.

All of experiments shown hereinabove were also repeated with another stem cell clone, named D16, with similar results, as illustrated in the following Example 3.

### EXAMPLE 3 Extracellular HMGB1, a signal of tissue damage, induces mesoangioblast migration and proliferation

### MATERIALS and METHODS

### Cells

Bovine Aorta Endothelial Cells (BAEC) were isolated from a section of the thoracic aorta of a freshly slaughtered calf as described (Palumbo al., Arterioscler. Thromb. Vasc. Biol., 22, 405-11, 2002). Mesoangioblasts were isolated from the dorsal aorta of mouse embryos and from juvenile arteries as previously described (De Angelis et al., J. Cell Biol., 147, 869-78, 1999). After cloning, cells were expanded on a feeder layer of mitomycin C-treated STO fibroblasts. Clones showing the mesoangioblast gene expression pattern (presence of CD34, Kit, Flk1 and MEF2D) were used for the in vitro and in vivo experiments. Embryonic mesoangioblasts (clone D16) were transduced with a lentiviral vector encoding for nuclear LacZ, while adult mesoangioblasts (clone G1) were labeled with DiI and then injected into the femoral artery of mice.

### HMGB1 and antibodies

Expression and purification of the full-length HMGB1 protein and fragments thereof was performed as described previously (Müller et al., Biochemistry, 40, 10254-10261, 2001). Endotoxins were removed by passage through Detoxy-Gel columns (Pierce).

Rabbit polyclonal anti-HMGB1 antibodies raised against the peptide 166-181 were from Pharmingen BD, polyclonal antibodies against box A were from MBL. The anti-RAGE goat antibody was from Chemikon. Western blots were performed as described (Degryse et al., J. Cell Biol., 152, 1197-2006, 2001).

### Proliferation assay

Cells were seeded in 6-well plates (1x10⁵ cells/well) and grown in RPMI supplemented with 20% FCS. After 24 hours the medium was replaced with serum-free RPMI for 16 hours. Subsequently the cells were grown with medium alone, or medium with the addition of 20% FCS or HMGB1 at the concentration of 1, 3, 10, and 30 ng/ml. Cells were counted after 1, 2 and 3 days, and Trypan blue dye exclusion was used as indicator of cell viability. All experiments were performed three times in duplicate.

### Chemotaxis assay

Cell migration was assayed using Boyden chambers (Degryse et al., J. Cell Biol., 152, 1197-2006, 2001). Briefly, PVP-free polycarbonate filters with 8 µm pores (Costar) were coated with 5 µg/ml porcine skin gelatin (Sigma). Serum-free RPMI (negative control), RPMI containing 10, 50 or 100 ng/ml HMGB1, and RPMI with 20% serum (positive control) were placed in the lower chambers. D16 cells were grown in RPMI plus 10% FCS, starved overnight, washed twice with PBS to eliminate any floating cells, and harvested with trypsin. Fifty thousand cells rsuspended in 200 µl RPMI were placed in the upper chambers and incubated at 37°C in 5% CO₂ for 16 h. Cells remaining on the upper surface of the filters were mechanically removed, those which had migrated to the lower surface were fixed with ethanol, stained with Giemsa Stain Modified (Sigma) and counted at 400x magnification in ten random fields per filter. Assays were performed in triplicate and repeated three times in independent experiments.

### Transmigration assay

BAEC cells were grown in DMEM plus 10% FCS on polycarbonate transwell inserts (3 µm pores; Costar) for 5 days until they formed a monolayer. The inserts were then placed between chambers in Boyden apparatuses, and the tightness of monolayers was checked by measuring the diffusion of BSA between chambers. Mesoangioblasts (10⁵ cells in 100 µl RPMI) were placed in the upper compartments and RPMI containing HMGB1 or VEGF (mature 121 amino acid variant of human VEGF expressed in *E. coli,* from R&D Systems) was placed in the lower compartments (500 µl). After 8 hours at 37°C the filters were removed, and the result was evaluated as described for the chemotaxis assay. These experiments were carried out three times in duplicate.

### Flow cytometry

Mesoangioblasts starved overnight were grown in RPMI medium alone, RPMI plus 20% FCS or RPMI plus 100 ng/ml HMGB1 for 6, 12, 24 or 48 hours. Cells were washed, fixed in 70% ethanol, stained with 50 µg/ml propidium iodide (PI) in PBS plus 50 µg/ml RNase A and incubated for 30 min at room temperature. The DNA content was measured by flow cytometry (FACScan; Becton-Dickinson) using the Standard CellQuest software.

### Estimation of the number of cell divisons

Ten million embryonic or adult mesoangioblasts were seeded on 100 mm dishes in RPMI supplemented with 20% FCS. After 24 hours the cells were starved in RPMI alone overnight. Cells were then washed in PBS, and CFDASE (Molecular Probes) was added to the final concentration of 2.5 µM for 8 minutes at room temperature. The staining was quenched by the addition of 10% FCS and cells were washed in RPMI. Fluorescently labeled cells were then grown in RPMI alone, RPMI plus 100 ng/ml HMGB1 or RPMI plus 20% FCS and harvested after 48 hours. Cells were then analyzed on FACScan.

### Cytoskeleton visualization

BAEC cells were grown on glass coverslips until fully confluent. After the treatments described in the text, the cells were washed with PBS and fixed with 4% paraformaldehyde at room temperature for 10 minutes. Cells were then stained with FITC-conjugated phalloidin (Sigma) to visualize the actin cytoskeleton as described (Degryse et al., J. Cell Biol., 152, 1197-2006, 2001).

### Preparation of HMGB1-loaded beads.

Heparin beads (34 µm diameter) were recovered from a HiTrap Heparin HP column (Pharmacia) and extensively washed in PBS. Beads (20 µl packed volume) were then incubated for 1 hour at 4°C with 60 µg HMGB1, harvested by centrifugation, washed twice with PBS and resuspended in PBS. SDS-PAGE was performed to check the amount of HMGB1 on the beads.

### Intra-artery delivery of mesoangioblasts in mice.

Heparin beads (a slurry containing 3 µg beads in 20 µl PBS), either laoded with HMGB1 or not, were injected with an insulin syringe into *tibialis anterior* muscles of 6-week old female CD-1 mice (3 per group). After 1 hour mesoangioblasts (4x10⁵ cells/animal) were injected through the femoral artery as previously described (Torrente et al., J. Cell Biol., 152, 335-48, 2001); animals were sacrified 24 hours later. For histochemistry analysis, samples of *tibialis anterior* muscles were frozen in liquid nitrogen-cooled isopentane and cryostat-sectioned. Serial muscle sections of 10 µm thickness were stained with X-gal for the experiment with LacZ labeled cells (Totsugawa et al., Cell Transplant., 11, 481-8, 2002), or visualized directly for the experiment with DiI. DiI was from Molecular Probes.

### RESULTS

### HMGB1 stimulates the proliferation of vessel-associated embryonic stem cells

Stem cells isolated from mouse dorsal aorta of E9.5 embryos (mesoangioblasts) were cultured in vitro and tested for the presence of the CD34, Kit, Flkl and MEF2D cellular markers (Minasi et al., Development, 129, 2773-2783, 2002). The authors used one of these clones (called D16) to assess whether HMGB1 can act as a mitogen.

D16 cells were seeded in RPMI medium with 20% FCS and then starved for 16 hours in the absence of serum to synchronize the cell population. Increasing concentrations of HMGB1 were then added to the medium without serum, and cells were counted after 1, 2, and 3 days. Figure 7A shows that there is a significant increase in the number of D16 mesoangioblasts after stimulation with HMGB1 up to day 2, while only slight proliferation occurs between days 2 and 3. All concentrations tested had similar effects. HMGB1-stimulated D16 cells had a normal morphology and excluded Trypan blue up to the end of the experiment, whereas cells in control cultures without HMGB1 were dying (not shown). HMGB1 has no mitogenic effect on 3T3 fibroblasts (Figure 7C).

The authors investigated in more detail the proliferative response of D16 cells to HMGB1: cells were exposed for 6, 12, and 24 hours to RPMI medium alone (negative control), or medium containing 30 ng/ml HMGB1 or 20% FCS, and analyzed for DNA content by FACS after propidium iodide staining. After six hours of stimulation with HMGB1, the majority of mesoangioblasts had entered the cell cycle; after 24 hours, most cells appeared to have a diploid DNA content and thus to be in G1 or G0 (Fig. 7B). The authors evaluated the number of cell cycles triggered by HMGB1 by staining at time 0 the cell membranes with the fluorescent dye CFDASE (Colombetti et al, J. Immunol., 169, 6178-86, 2002)6; after 48 hours most cells had one-half of the initial quantity of dye (and so had undergone 1 division) and a minority had one-quarter (and had undergone two divisions). By comparison, all cells cultured in 20% FCS had divided at least twice (data not shown).

These data indicate that HMGB1 induces a limited number of cell divisions. This might be due to a specific program of mesoangioblasts, or to the depletion of HMGB1 in the medium. The authors therefore added 30 ng/ml HMGB1 at time 0, and additional HMGB1 at 12, 36 and 60 hours. Mesoangioblasts continually exposed to HMGB1 continued to divide (Figure 8). After 48 hours, no HMGB1 was detectable by Western blot in the medium of cells stimulated once, while HMGB1 equivalent to about 40 ng/ml remained in the medium of multiply-exposed cells (see inset panel Fig 8). Taken together these results indicate that HMGB1 acts as a growth factor for D16 cells, but is rapidly depleted.

### HMGB1 induces mesoangioblast migration

The authors have previously shown that HMGB1 is a chemoattractant for rat smooth muscle cells (RSMC) (Degryse et al. , J. Cell Biol., 152, 1197-2006, 2001). They then investigated whether HMGB1 is a chemoattractant for D16 cells too. In a chemotaxis assay using modified Boyden chambers, HMGB1 stimulated migration of D16 cells in a concentration-dependent manner (Fig. 9A). Polyclonal antibodies raised against amino acids 166-181 of HMGB1 (anti 166-181, Fig 7A), significantly reduced the migratory response. However, the migratory response was unaffected by anti-HMGB1 monoclonal antibodies that recognize box A.

The authors also tested the individual domains of HMGB1 for the ability to induce D16 cell migration (Fig. 9B). Neither box A nor box B alone induced appreciable migration. The didomain fragment (comprising boxes A and B) had a non-significant effect, whereas the ABbt fragment, that only lacks the acidic tail (Fig. 9C), was as potent as the full-length protein.

Huttunen et al. (Cancer Res., 62, 4805-4811, 2002) have identified residues 150-183 as the HMGB1 segment that interacts with RAGE. Remarkably, the anti-HMGB1 antibodies that block D16 migration (Fig. 9A) specifically interact with the amino acid stretch between residues 166 and 181 (Fig. 9C), and therefore occude the RAGE-interacting surface. The monoclonal antibodies that recognize box A cannot prevent the interaction with RAGE. Our data therefore indicate that HMGB1 is a powerful chemoattractant for D16 cells, and suggest that RAGE is its receptor. RNA profiling of D16 cells (not shown) indicate that they express RAGE, and RAGE protein is detectable in D16 cells by Western blot (Fig. 9D).

### HMGB1 induces mesoangioblast migration across endothelial monolayers

Mesoangioblasts are vessel-associated stem cells that can migrate to damaged tissues through the general circulation (Sampaolesi et al., unpublished data), and have the ability to transit through the endothelial barrier. The authors then tested whether HMGB1 could also promote the transmigration of stem cells across an endothelial monolayer grown on the septum between the chambers of a Boyden apparatus. When the authors added 100 ng/ml HMGB1 to the medium in the lower chamber, the number of D16 cells crossing the monolayer increased 8-fold when compared to medium without any addition (Fig. 10B). HMGB1 has higher potency than vascular endothelial growth factor (VEGF), a signaling molecules is known to promote cell migration across endothelial barriers. VEGF induces profound cytoskeletal reorganization of endothelial cells, characterized by the formation of transcytoplasmic stress fibers and the disassembly of adherens junctions, which are important to maintain the endothelial barrier function (Rousseau et al., J. Biol. Chem., 275, 10661-72, 2000; Esser et al., J. Cell Sci., 111, 1853-65, 1998). Thus, the authors compared the effect of HMGB1 on endothelial cells to that of VEGF.

After stimulation with HMGB1 for 5 to 30 minutes, endothelial cells were fixed, labeled with fluorescein-conjugated phalloidin and examined by immunofluorescence. Both HMGB1 and VEGF caused stress fiber formation and disaggregation of endothelial cells, suggesting that HMGB1 can dramatically increase the permeability of the endothelial lining of vessels (Fig. 10A).

### HMGB1 directs mesoangioblast homing in vivo

The authors finally assessed HMGB1's ability to control mesoangioblast migration *in vivo.* Heparin beads were loaded with HMGB1 at the concentration of 3 µg/ml and then injected with a fine needle into the *tibialis anterior* muscle of mice. D16 cells transduced by a lentiviral vector causing the expression of nuclear LacZ were injected after 30 min through the proximal femoral artery (see materials and methods). The mice were sacrificed after 24 hours and the *tibialis anterior* muscle was removed, sectioned, and analyzed by immunohistochemistry. Muscles injected with HMGB1-loaded beads showed a considerable swelling compared to both sham-injected muscles (not shown) and muscles injected with unloaded heparin beads (Fig. 11A), suggesting that HMGB1 caused considerable muscle inflammation. This is consistent with HMGB1's role as proinflammatory cytokine.

Muscle sections were stained with X-gal and blue cells were scored using computer-assisted imaging techniques (Fig. 11). Large groups of blue cells were found in the vicinity of HMGB1-loaded beads (panel C); a minority of sections displayed individual blue cells dispersed throughout the muscle (panel D). The sections from muscles injected with unloaded beads had no blue cells at all (panel B).

These observations clearly suggest that HMGB1 is able to recruit mesoangioblasts in vivo.

### The biological action of HMGB1 on adult mesoangioblats

These findings identify a role for extracellular HMGB1, a proinflammatiory cytokine, on the migration and proliferation of embryonic mesoangioblasts. However, inflammation does not occur in the embryo even in the presence of tissue damage. The authors then asked whether HMGB1 had an effect on adult mesoangioblasts as well.

The authors repeated the experiments described previously on adult mesoangioblasts isolated from bone marrow (G1 clone, see materials and methods). Figure 12 summarizes our findings. HMGB1 causes adult stem cell proliferation (Panel A), chemotaxis and transmigration (panel B). Finally, like embryonic mesoangioblasts, adult mesoangioblasts can be recruited by HMGB1 into the *tibialis anterior* muscle (Fig 12C).

Similar effects were also observed in additional experiments with different adult mesoangioblast lines derived from the aorta of 8-week old mice (data not shown).

### EXAMPLE 4

### MATERIALS AND METHODS

### Immunoistochemical analysis

Hearts were arrested in diastole at 1 week from the infarction, perfused retrogradely with 10% (vol/vol) formalin, embebbed in paraffin and sectioned at 4 µm. Sections were stained with hematoxilin and eosin (H&E) for histologic examination or processed for immunoistochemical analysis to identify newly formed cardiac cells. The following antibodies were used to assess cardiac differentiation: rabbit polyclonal connexin 43 antibody (Sigma St. Louis, MO, USA), mouse monoclonal anti α-sarcomeric actin (clone 5C5; Santa Cruz Biotechnology, Santa Cruz, CA, USA), rabbit polyclonal anti-MEF-2 (C-21; Santa Cruz). Ki67 was detected by using mouse monoclonal antibody Ki67 (clone MIB5 Novocastra Laboratories, UK). FITC conjugated goat anti-rabbit and TRITC conjugated goat anti mouse (Sigma) were used as secondary antibodies.

### Evaluation of myocardial function

Echocardiography was performed in conscious mice with a Sequoia 256c instrument equipped with a 13-MHz linear transducer. Two dimensional images and M-mode tracings were recorded from the parasternal short axis view at the level of papillary muscle. From M-mode tracings, anatomical parameters in diastole and systole were obtained (Orlic et al., Nature, 98, 10344-10349, 2001). For hemodynamic studies, mice were anesthetized with chloral hydrate (400 mg/kg body weight), the right carotid artery was cannulated with a microtip pressure transducer (Millar 1.4F) and the Left Ventricular pressures (LV, LV+ and - dP/dt), were measured.

### RESULTS

### HMGB1 promotes the formation of new myocytes in the infarcted heart

Myocardial infaction was induced in C57BL/6 mice by ligating the left coronary artery (Li et al., J. Clin. Invest., 100, 1991-1999, 1997) under anesthesia. After 4 hrs animals were re-operated and 200 ng of purified HMGB1 was administered in the peri-infarcted area in the left ventricle. Control animals consisted of infarcted mice injected with 200 ng of an unrelated protein (GST).

Injection of HMGB1 in the peri-infarcted left ventricle results in the formation of a compact tissue characterized by aligned cells occupying most of the damaged area, as showed by hematoxylin and eosin staining (Fig. 13A). The newly developed tissue extended from the border zone, where it was more compact and organized, to the interior of the injured region. When a control protein (GST) was used in similar experiments, new tissue consisting of aligned cells was never observed.

To characterize the newly formed tissue, the authors probed the expression of α-sarcomeric actin, a specific marker of cardiac differentiation. Immunoistochemical analysis showed that the tissue present in HMGB1-injected hearts (n=8) consists of α-sarcomeric actin positive cells (Fig 13C). Moreover some cells expressed connexin 43, a component of gap-junctions between cardiomyocytes (not shown). Interestingly, α-sarcomeric actin expressing cells were also positive for MEF2, a transcription factor involved in the activation of the promoters of several cardiac structural genes (Fig 13D). Conversely, only infiltrating cells were observed in GST-treated hearts (n=6). The authors also evaluated the growth stage of the cells in the HMGB11 treated heart, by analyzing the expression of Ki67, a protein that is present in G1, S, G2 and early mitosis. Ki67 was expressed in some newly-formed cells, localized inside the infarcted tissue (Fig. 13B). These data demonstrate that HMGB1 promotes the formation of new myocytes in the infarcted heart.

### Myocardial function after infaction is improved by HMGB1

One week following infarction, the authors investigated echocardiographic and hemodynamic parameters in the surviving mice. In comparison with sham-operated mice, the infarcted animals whether treated or not with HMGB1 showed indices of cardiac failure. In comparison to infacted but untreated mice, however, mice treated with HMGB1 showed a significant recovery of cardiac performance. Ejection Fraction (EF) was 51% higher in HMGB1 treated than in GST treated mice (Fig. 14). LV end diastolic pressure (LVEDP) was 39% lower than in GST treated mice, reaching levels similar to those obtained in sham-operated mice (Fig. 15). The changes in LV developed pressure (LVDP) and + and - dP/dT were also improved in HMGB1-treated mice showing an increase 34%, 39% and 45% above control, respectively.

Therefore HMGB1, by inducing cardiomyocytes formation, improved left ventricular performance in the infarcted heart.

## Claims

1. Use of a HMGB1 protein or functional parts thereof, or of a HMGB1 expressing vector, for the preparation of a medicament for the treatment of tissue damage and/or to promote tissue repair and regeneration wherein the tissue regeneration depends on the growth of cells of the same type as those damaged, excluding connective tissue.

2. Use according to claim 1 wherein the tissue is cardiac or skeletal muscle.

3. Use according to claim 1 wherein the tissue repair and/or regeneration includes the repair and/or regeneration of areas of necrosis.

4. Use according to claim 3 wherein the areas of necrosis comprise trauma sites, ischemia sites including infarcted heart, burn sites.

5. Use according to any of previous claims wherein the medicament further comprises an effective amount of an anti-inflammation agent.

6. Use according to any of previous claims wherein the medicament further comprises diluents and/or adjuvants and is administered by perfusion at the tissue repair site.

7. Use according to claim 1 to 5 wherein the medicament further comprises diluents and/or adjuvants and/or carriers and is administered by intramuscular injection.

8. Use according to any of the previous claim wherein the medicament further comprises adult or non-human embryonic stem cells.

9. Use according to claim 8 wheren said stem cells are mesoangioblasts.

10. Method to promote adult or non-human embryonic stem cell migration and/or proliferation in cell culture comprising the step of exposing such cells to an effective amount of the HMGB1 protein or functional parts thereof.

11. Method according to claim 10 wherein said stem cells are resident cardiac or circulating stem cells.

12. Method to promote the proliferation of cardiomyocytes in cell culture comprising the step of exposing such cells to an effective amount of the HMGB1 protein or functional parts thereof.

## Patentansprüche

1. Verwendung eines HMGB1 Proteins oder funktioneller Teile davon, oder eines HMGB1 Expressionsvektors für die Herstellung eines Medikaments zur Behandlung von Gewebeschäden und/oder zur Förderung einer Gewebereparatur und -regeneration, wobei die Geweberegeneration von dem Wachstum von Zellen derselben Art wie der geschädigten abhängig ist, ausschließlich Bindegewebe.

2. Verwendung nach Anspruch 1, wobei das Gewebe Herz- oder Skelettmuskel ist.

3. Verwendung nach Anspruch 1, wobei die Gewebereparatur und/oder -regeneration die Reparatur und/oder Regeneration von Nekrosearealen beinhaltet.

4. Verwendung nach Anspruch 3, wobei die Nekroseareale Traumabereiche, Ischämiebereiche einschließlich infarzierter Herz-, Verbrennungsbereiche umfassen.

5. Verwendung nach einem der vorangehenden Ansprüche, wobei das Medikament des Weiteren eine wirksame Menge eines entzündungshemmenden Mittels umfasst.

6. Verwendung nach einem der vorangehenden Ansprüche, wobei das Medikament des Weiteren Verdünnungsmittel und/oder Adjuvantien umfasst und durch Perfusion an der Gewebereperaturstelle verabreicht wird.

7. Verwendung nach einem der Ansprüche 1 bis 5, wobei das Medikament des Weiteren Verdünnungsmittel und/oder Adjuvantien und/oder Träger umfasst und durch intramuskuläre Injektion verabreicht wird.

8. Verwendung nach einem der vorangehenden Ansprüche, wobei das Medikament des Weiteren erwachsene oder nicht humane embryonale Stammzellen umfasst.

9. Verwendung nach Anspruch 8, wobei die Stammzellen Mesoangioblasten sind.

10. Verfahren zur Förderung der Migration von erwachsenen oder nicht humanen embryonalen Stammzellen in Zellkultur, umfassend den Schritt des Belastens solcher Zellen mit einer wirksamen Menge des HMGB1 Proteins oder funktioneller Teile davon.

11. Verfahren nach Anspruch 10, wobei die Stammzellen residente Herz- oder zirkulierende Stammzellen sind.

12. Verfahren zur Förderung der Proliferation von Cardiomyozyten in Zellkultur, umfassend den Schritt des Belastens solcher Zellen mit einer wirksamen Menge des HMGB1 Proteins oder funktioneller Teile davon.

## Revendications

1. Utilisation d'une protéine HMGB1 ou de parties fonctionnelles de celle-ci, ou d'un vecteur exprimant HMGB1, pour la préparation d'un médicament destiné au traitement d'une lésion tissulaire et/ou destiné à la promotion de la régénération et de la réparation tissulaires, la régénération tissulaire dépendant de la croissance de cellules du même type que celles endommagées, à l'exclusion du tissu conjonctif.

2. Utilisation selon la revendication 1, dans laquelle le tissu est un muscle cardiaque ou squelettique.

3. Utilisation selon la revendication 1, dans laquelle la réparation et/ou la régénération tissulaires comprennent la réparation et/ou la régénération de zones de nécrose.

4. Utilisation selon la revendication 3, dans laquelle les zones de nécrose comprennent des sites de traumatisme, des sites d'ischémie comprenant un coeur ayant subi un infarctus, des sites de brûlure.

5. Utilisation selon l'une quelconque des revendications précédentes, dans laquelle le médicament comprend en outre une quantité efficace d'un agent anti-inflammatoire.

6. Utilisation selon l'une quelconque des revendications précédentes, dans laquelle le médicament comprend en outre des diluants et/ou des adjuvants et est administré par perfusion au niveau du site de réparation tissulaire.

7. Utilisation selon les revendications 1 à 5, dans laquelle le médicament comprend en outre des diluants et/ou des adjuvants et/ou des vecteurs et est administré par injection intramusculaire.

8. Utilisation selon l'une quelconque des revendications précédentes, dans laquelle le médicament comprend en outre des cellules souches embryonnaires non humaines ou adultes.

9. Utilisation selon la revendication 8, dans laquelle lesdites cellules souches sont des mésoangioblastes.

10. Procédé de promotion de la migration et/ou de la prolifération des cellules souches embryonnaires non humaines ou adultes en culture, qui comprend l'étape consistant à exposer ces cellules à une quantité efficace de protéine HMGB1 ou de parties fonctionnelles de celle-ci.

11. Procédé selon la revendication 10, dans lequel lesdites cellules souches sont des cellules souches circulantes ou cardiaques résidentes

12. Procédé de promotion de la prolifération des cardiomyocytes en culture cellulaire, qui comprend l'étape consistant à exposer ces cellules à une quantité efficace de protéine HMGB1 ou des parties fonctionnelles de celle-ci.
